# EUROPEAN PATENT APPLICATION

(11) **EP 2 653 870 A1**
(43) Date of publication of application: **23.10.2013**
(21) Application number: 12305449.6
(22) Date of filing: 17.04.2012
(51) Int. Cl.: G01N 33/574, G01N 33/68

(54) **Method for the selection of serum biomarkers of epigenetic alterations, particularly of global hypomethylation and their uses**

(71) Applicant: UNIVERSITE PARIS DESCARTES, 75006 Paris (FR)
(72) Inventor: Bellet, Dominique, 92100 Boulogne-Billancourt (FR); Pecking, Alain, 92700 Colombes (FR); Richon, Sophie, 95290 L'Isle Adam (FR)
(74) Representative: Regimbeau

(57) **Abstract**

The present invention provides a novel method for the selection of serum biomarkers of epigenetic alterations, particularly of global hypomethylation and the use of said biomarkers in a method for diagnosing pathology associated to epigenetic alterations of cell in an individual, such as placental-related pathology or cancer. The present invention also relates to a method of detecting a predisposition to placental-related pathology or cancer based on the presence or the level of said biomarker in a serum or plasma sample of said patient. The present invention is directed to a kit comprising such serum biomarkers of epigenetic alterations, particularly of global hypomethylation.

## Description

The present invention provides a novel method for the selection of serum biomarkers of epigenetic alterations, particularly of global hypomethylation and the use of said biomarkers in a method for diagnosing pathology associated to epigenetic alterations of cell in an individual, such as placental-related pathology or cancer. The present invention also relates to a method of detecting a predisposition to placental-related pathology or cancer based on the presence or the level of said biomarker in a serum or plasma sample of said patient. The present invention is directed to a kit comprising such serum biomarkers of epigenetic alterations, particularly of global hypomethylation.

Although the cells in the human body contain the same DNA sequence, the function and phenotype differ [1, 2]. This implies that, apart from genetic programming, the phenotype is regulated by another phenomenon. This is called epi- (greek for upon, above)-genetic. Epigenetic inheritance is defined as cellular information, other than the DNA sequence itself, that is heritable during cell divisions. Epigenetic templates that control gene expression are transmitted to daughter cells independently of the DNA.sequence. There are three main, inter-related types of epigenetic inheritance: DNA methylation, genomic imprinting and histone modification [3]. It has become increasingly apparent that epigenetic inheritance is important in many physiological and pathophysiological conditions. Epigenetic codes are much more subject to environmental influences than the DNA sequence. This could explain how lifestyle and toxic chemicals affect susceptibility to diseases. Up to 70% of the contribution to a particular disease can be nongenetic [4].

Epigenetic templates can change and be alterated during normal ageing and contribute to common cancer risk in adults. The single leading risk factor for cancer is age. Although that has often been attributed to the accumulation of mutations over time, an alternative and complementary interpretation is that age itself disrupts the epigenetic programme, increasing cancer risk. Epigenetic alterations can also support clonal evolution in human cancers, contributing to tumour progression. Thus, epigenetics play key role in cancer development and come to rival genetics.

Amongst epigenetic alterations in cancer, DNA methylation alterations are divided by the contrary findings of increased methylation (hypermethylation) at specific gene promoters within a genomic environment of a general loss of methylation (hypomethylation) compared to normal cells. Cancer-associated DNA hypomethylation is as prevalent as cancer-linked hypermethylation, but these two types of epigenetic abnormalities usually seem to affect different DNA sequences. DNA hypomethylation in cancer often affects more of the genome than does hypermethylation so that net losses of genomic 5-methylcytosine (m⁵C) are seen in many human cancers [5] .

The discovery of extensive cancer-associated DNA hypomethylation in the human genome preceded that of cancer-linked DNA hypermethylation. However, until recently, there has been much emphasis on the critical role of DNA hypermethylation in human carcinogenesis. In comparison, there has been inadequate attention paid to decreased methylation of DNA in cancer and hypomethylation in cancer is only now going a renaissance [6]._Genome wide hypomethylation is a decrease (compared to total cytosines) from approximately 4% in most normal tissues to 2-3% in cancer tissues [7]. This change in cancer tissues was first observed in lung and colon carcinomas compared to adjacent normal tissues and in various malignancies compared to various postnatal tissues, demonstrating that overall genomic methylcytosine levels were lower in cancer tissues [8, 9]. Moreover, high-throughput genomic methylation analysis of tumours [10, 11], including cancers of the stomach, kidney, colon, pancreas, liver, uterus, lung and cervix [12-19] show that the frequency of hypomethylated is quite high. Finally, high percentage of malignant tumors, especially metastases, have DNA with unusually low methylcytosine contents relative to the normal tissues.

The high frequency of cancer associated DNA hypomethylation, the nature of the affected sequences, and the absence of associations with DNA hypermethylation are consistent with an independent role for undermethylation of certain DNA sequences in cancer formation or tumor progression. In addition, hypomethylation is a mechanism of drug, toxin and viral effects in cancer and an exciting development in cancer hypomethylation involves a link to diet.

Genomic hypermethylation in cancer has been observed most often in CpG islands in gene regions. In contrast, very frequent hypomethylation is seen in both highly and moderately repeated DNA sequences in cancer. The magnitude of the decrease in methylation observed in cancers compared to matched controls (4% down to 2-3% total methyl-cytosine) is most commonly accounted for by loss of methylation at repetitive sequences [20].

Amongst highly repetitive DNA sequences that display tumor-associated hypomethylation are endogenous retrotransposons [6]. Retrotransposons, also called transposons via RNA intermediates, are genetic elements that can amplify themselves in a genome. Retrotransposons consist of two sub-types, the long terminal repeat (LTR) and the non-LTR retrotransposons. Around 42% of the human genome is made up of retrotransposons. Amongst those retrotransposons that display tumor-associated hypomethylation are Long Interspersed Nuclear Element 1 (LINE-1) retrotransposons, which are 6-kb interspersed DNA repeats. LINE-1 are non-LTR retrotransposons which makes up around 15% of the human genome [21-23].

Amongst moderately repeated DNA sequences, there is the other class of human retrotransposons that display tumor-associated hypomethylation, the LTR retrotransposons including those from endogenous retroviruses, especially, the HERV-K family [6]. While there are only about 30 ± 50 full-length HERV-K sequences in the human genome, there are an estimated 10 000 solitary long terminal repeats (LTRs) from HERV-K [24]. At least some of the solitary LTRs can drive reporter gene expression.

Retrotransposons or retroviral derived elements can have their transcription upregulated in vivo by DNA demethylation in both cancer and placental cells. As representative examples, high-level expression of HERV-K gag and env RNA is associated with germ cell and trophoblastic tumors [25]. Moreover, env RNA was detected in most assayed term placental samples ([7, 26]. This latter observation may be related to the facts that much of term placental tissue is of trophoblast origin and that placental DNA displays global genomic hypomethylation. Indeed, genome wide hypomethylation decreases from approximately 4% in most normal tissues to 0.76% in normal placenta [7].

Another type of repeated DNA sequence is satellite DNAs which are tandem, high-copy-number repeats composed of variations of oligonucleotide. The hypomethylation of repeated DNA sequences may play special role in carcinogenesis such as increasing karyotype instability or affecting gene expression. Thus, for some type of cancer, DNA hypomethylation is seen as an early indicator of tumorigenesis [27, 28].

In addition, single copy sequences in the genome also exhibit hypomethylation in cancer, often associated with increased expression. Several tumor- or proliferation-associated genes have been found to be hypomethylated in human cancers. Examples of genes that are affected by hypomethylation include oncogenes such as HRAS and the Cancer Testis (CT) genes which encode Cancer Testis antigens [3]. Those CT genes are expressed normally in both the testis and aberrantly in tumours. Interestingly, they are also expressed normally in the placenta [29]. Indeed, hypomethylation of various DNA sequences in cancers and placenta seems to be a common theme. As previously indicated, global genomic hypomethylation of DNA is observed in trophoblastic cells, the constituent cells of the placenta, and in tumorigenic or metastatic cancer cells [7]. This observation at the epigenomic level might be in line with the fact that placental and cancer cells share molecular circuits which are implicated in the proliferative, invasive and migratory properties of these cells [29-31]. Indeed, an overview of signalling circuitries used by trophoblast cells places emphasis on the many circuitries shared with those employed by cancer cells. As virtually all mammalian cells carry similar molecular machinery regulating their proliferation, differentiation and death and as most regulatory and effector components are present in a redundant form, it is not totally surprising that normal trophoblasts and malignant cells, which may have to accomplish comparable tasks to proliferate and migrate so as to ultimately invade neighbouring tissue, use, in part, similar regulatory and effector components, similar circuitries and similar mechanisms governed by similar epigenetic templates [29].

Finally, the high frequency of cancer-linked DNA hypomethylation, the nature of the affected sequences, and the absence of associations with DNA hypermethylation are consistent with an independent role for DNA undermethylation in cancer formation or tumor progression. Hypomethylation of repeated or single-copy DNA sequences has been significantly correlated with disease progression for some tumors [22, 27, 32]. Furthermore, a number of studies have shown significant decreases in global DNA methylation levels with progressing tumor stage, tumor grade, or various other indicators of poor prognosis [33, 34], and global DNA hypomethylation in cancer is significantly associated with hypomethylation of certain DNA repeats [27, 28, 35]. In addition, for some types of cancer, DNA hypomethylation is seen as an early indicator of tumorigenesis. As a consequence in clinical practice, DNA hypomethylation analysis may be useful in detecting cancer and managing the disease [6].

Similarly, DNA hypomethylation analysis may also be useful for the screening of abnormal pregnancies. A significant factor in placental development and function is epigenetic regulation. Disturbed placental epigenetics has been demonstrated in human placental-related pathologies like small for gestational age (SGA), intrauterine growth retardation (IUGR) and pre-eclampsia [1].

Therefore, assays for DNA hypomethylation may become a clinically useful addition to hypermethylation analyses of CpG islands.

The range of methods currently available for the study of genomic methylation has expanded considerably since initial studies in the field [36]. Traditionally, methyl-cytosine content was measured using high performance liquid chromatography (HPLC) of genomic DNA digested to individual deoxynucleosides [9, 37, 38]. Although this method gives an absolute quantitative measure of methylcytosine content, it is time consuming and requires a large amount of DNA. Several alternative methods have since been developed, including Southern blot [8], Methyl group acceptance assay (MAA) [39]; direct bisulphite sequencing [40]; methylation sensitive PCR (MSP) and quantitative PCR (qPCR) based method called MethyLight [41] and lastly, monoclonal antibodies against 5-methylcytosine have been developed and used to reliably stain methylated DNA in vitro..

Using these methods, epigenetic alterations have been detected in plasma and serum These alterations appear to be valuable molecular biomarkers and have been studied at the molecular level in either circulating tumor DNA or circulating tumor cells [42-47]. Methods for detecting these molecular biomarkers have the key advantage of being noninvasive but are not really simple and do not fit well with a clinical utilization on a routine basis.

Currently there is a need to provide biomarkers of epigenetic alterations and/or global hypomethylation, which can be detecting by non-invasive methods and which are simple to implement, such as biomarkers of epigenetic alterations which can be detected in serum or plasma at the peptide and protein biomarkers, and which can be thus used with a clinical utilization on a routine basis.

This is the object of the present invention.

The invention relates to a novel concept for the detection of epigenetic alterations in cancer and/or placental related pathologies which is to detect biomarkers of epigenetic alterations in serum or plasma at the peptide and protein levels. This Concept of novel tests measuring serum Biomarkers of epigenetic alterations (CB tests) is also designated SEBEA tests for SErum Biomarkers of Epigenetic Alterations tests (SEBEA tests ).

Surprisingly, the inventors have demonstrated that epigenetic alterations and, in particular, the global hypomethylation of both cancer and placental cells may ultimately lead to the expression of peptides and proteins which could be shed in the blood stream. These peptides and proteins may constitute surrogate biomarkers of global hypomethylation. The presence of these peptides or proteins would be a witness of the altered epigenetic templates of either cancer cells or abnormal trophoblastic cells and the sensitive and specific detection of these surrogate biomarkers of global hypomethylation might constitute novel non-invasive methods for the detection or the management of cancer and placental related diseases.

As both trophoblastic cells and tumorigenic or metastatic cancer cells display global hypomethylation, the inventors have suggested that peptides or proteins expressed by trophoblastic cells as a consequence of their global hypomethylation might also be expressed by cancer cells and be present at increased levels in the blood stream of both cancer patients and pregnant women with placental-related pathologies. This is what it has been demonstrated by the inventors.

Thus, in a first aspect, the present invention is directed to a method for selecting peptides and proteins that can serve as biomarkers of epigenetic alterations, particularly of global hypomethylation, in the serum or plasma, said method comprising the step of selecting peptides or proteins which are present in the blood stream, such as serum or plasma, as a consequence of global hypomethylation of both trophoblastic cells and/or tumorigenic or metastatic cancer cells.

In a preferred embodiment, the present invention concerns a method for the selection of a serum biomarker of epigenetic alterations, particularly of global hypomethylation, said method comprising the steps of:
a) selecting a gene which is expressed (more preferably protein or peptide expression) in a cancer and/or a placental cell, preferably both in a cancer cell and in a placental cell, and whose expression in said cell is due to the hypomethylation of its expression regulation system/promoter;
b) optionally, determining the presence or the increase of the protein, or a specific fragment thereof, encoded by said gene selected in step a) in a serum or plasma sample of a patient known to exhibit a cancer or a placental-related pathology, preferably compared to a healthy patient serum or plasma sample; and
c) selecting said protein, or a specific fragment thereof, encoded by said gene as a serum biomarker of epigenetic alteration and, optionally, of global hypomethylation, whether this gene satisfy the step a) and, optionally, step b) selection.

In a preferred embodiment, said placental cell is a trophoblastic cell.

In an also preferred embodiment, said cancer cell is selected from tumorigenic or metastatic cancer cell.

In a preferred embodiment, said cancer is selected from breast, bladder, lung or colorectal cancer.

In another embodiment, the present invention is also directed to a method for the selection of a serum biomarker of epigenetic alterations, particularly of global hypomethylation, said method comprising the steps of:
a) selecting a gene which is expressed (more preferably protein or peptide expression) in a cancer and/or a placental cell, preferably both in a cancer cell and in a placental cell, said cell exhibiting a global hypomethylation and, optionally, the expression of said gene in said cell being due to the said global hypomethylation, particularly the hypomethylation of the regulation system/promoter of said gene expression;
b) optionally, determining the presence or the increase of the protein, or a specific fragment thereof, encoded by said gene selected in step a) in a serum or plasma sample of a patient known to exhibit a cancer or a placental-related pathology, preferably compared to a healthy patient serum or plasma sample; and
c) selecting said protein, or a specific fragment thereof, encoded by said gene as a serum biomarker of epigenetic alterations, particularly of global hypomethylation, whether this gene satisfy the step a) and, optionally, step b) selection.

Methods to measure Global DNA methylation or gene-specific methylation are well known by the skilled person.

Among the methods which can be used for determining whether the cell exhibits a global hypomethylation or whether the gene expression regulation system or promoter is hypomethylated (see optional step b) of the above method) the following methods and references can be cited:
- Methyl-cytosine content measure using high performance liquid chromatography (HPLC) of genomic DNA digested to individual deoxynucleosides [9, 37, 38];
- Southern blot [8];
- Methyl group acceptance assay (MAA) [39];
- Direct bisulphite sequencing [40] which can be adapted to assay repetitive elements using degenerate primers to conserved sequences such as LINE1 and can be accurately quantified using Pyrosequencing [48];
- Methylation sensitive PCR (MSP) and quantitative PCR (qPCR) based method called MethyLight [41]; and lastly,
- Monoclonal antibodies against 5-methylcytosine which have been developed and used to reliably stain methylated DNA in vitro. This has been applied to immunohistochemistry [49], immunofluorescence [50], flow cytometry [51] and DNA immunoprecipitation combined with microarrays or high throughput sequencing [52, 53].

Methods for DNA methylation analysis can be divided roughly into two types: global and gene-specific methylation analysis. For global methylation analysis, there are methods which measure the overall level of methyl cytosines in genome such as chromatographic methods and methyl accepting capacity assay. For gene-specific methylation analysis, a large number of techniques have been developed. Most early studies used methylation sensitive restriction enzymes to digest DNA followed by Southern detection or PCR amplification. Recently, bisulfite reaction based methods have become very popular such as methylation specific PCR (MSP), bisulfite genomic sequencing PCR. Additionally, in order to identify unknown methylation hot-spots or methylated CpG islands in the genome, several of genome-wide screen methods are also available such as Restriction Landmark Genomic Scanning for Methylation (RLGS-M), and CpG island microarray.

The term "patient" or "individual" includes mammals, e. g., humans, dogs, cows, horses, kangaroos, pigs, sheep, goats, cats, mice, rabbits, rats, and transgenic non-human animals, human is preferred.

Samples for use in the assays of the invention can be obtained by standard methods including particularly venous puncture.

In another aspect, the present invention concerns a method for diagnosing pathology associated to epigenetic alterations, particularly to global hypomethylation, of cell in a patient comprising the steps of:
i) selecting at least one serum biomarker of epigenetic alterations, particularly of global hypomethylation, selected by the method of the present invention, whose cell specific expression is due to said global hypomethylation, particularly to the hypomethylation of the system/promoter which regulates its expression;
ii) optionally, determining the methylation status, preferably the hypomethylation, of said regulation system, or part thereof, in a sample from said patient;
iii) determining the presence or the level of said biomarker selected in step a) in a serum or plasma sample of said patient,
   wherein the significant presence or the increase of said biomarker in said patient sample, preferably compared with said biomarker level found for healthy patient serum or plasma sample, is indicative of said pathology.

In a preferred embodiment, said pathology associated to epigenetic alteration is a cancer or a placental-related pathology.

In a preferred embodiment, said placental-related pathology.is selected from the group consisting of small for gestational age (SGA), intrauterine growth retardation (IUGR), pre-eclampsia and chromosomally abnormal pregnancy (such as Down syndrome, Trisomies 13 and 18, Klinefelter syndrome or XYY abnormality, preferably Down syndrome).

In a preferred embodiment, said cancer is selected from tumorigenic or metastatic cancer.

In a preferred embodiment, said cancer is selected from breast, bladder, lung or colorectal cancer.

In another aspect, the present invention is directed to a method for detecting a predisposition to, or the incidence of, placental-related pathology or cancer pathology in a patient which comprises:
i) selecting at least one serum biomarker of epigenetic alterations and, optionally, of global hypomethylation selected by the method of claim 1;
ii) determining the presence or the level of said biomarker selected in step a) in a serum or plasma sample of said patient,
   wherein the significant presence or the increase of said biomarker in said patient sample, preferably compared with the level of said biomarker found for healthy patient serum or plasma sample, is indicative of a predisposition to, or the incidence of, placental-related pathology or cancer.

By significant increase, it is intended to mean that the level of the biomarker found in the serum or plasma sample of the patient to be tested, is at least 1.5 times, preferably twice, more preferably 3, 5 or 10 times higher than the level obtained for healthy patient.

In a preferred embodiment, in the method for detecting a predisposition to, or the incidence of, placental-related pathology or cancer pathology in a patient according to the present invention, said pathology associated to epigenetic alteration is a cancer or a placental-related pathology is selected from the group consisting of small for gestational age (SGA), intrauterine growth retardation (IUGR), pre-eclampsia and chromosomally abnormal pregnancy and/or said cancer is selected from tumorigenic or metastatic cancer, more preferably selected from breast, bladder, lung or colorectal cancer.

In a preferred embodiment, said expression regulation system is selected from promoter or retrotransposon expression regulation system.

In another aspect, the present invention is directed to a method for diagnosing pathology associated to epigenetic alterations, particularly to global hypomethylation, of cell in a patient, or to a method for detecting a predisposition to, or the incidence of, placental-related pathology or cancer pathology in a patient according to the present invention, wherein in step ii) or iii), the determination of the presence or the level of said biomarker in a serum or plasma sample of said patient is carried out by the use of antibody specifically directed against the selected serum biomarker of epigenetic alterations, particularly of global hypomethylation.

In a particular aspect, the present invention is directed to a method for diagnosing pathology associated to epigenetic alterations, particularly to global hypomethylation, of cell in a patient, or to a method for detecting a predisposition to, or the incidence of, placental-related pathology or cancer pathology in a patient according to the present invention, wherein said at least one serum biomarker of epigenetic alterations, particularly of global hypomethylation, is selected from the group consisting of the INSL4 (pro-EPIL) and hCGβ type II peptide (or protein) biomarker.

In the present description, peptide, polypeptide or protein are used interchangeably and have the same meaning.

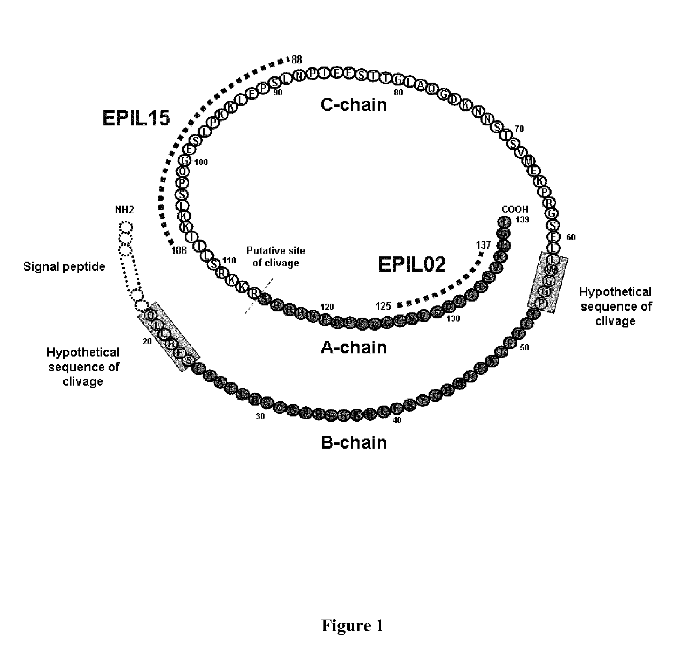

EPIL is a 139-amino-acid polypeptide (which is synthesized as a preprohormone characterized by a signal peptide, a B-chain, a connecting C-peptide and a terminal A-chain. In placenta, it was found that trophoblast cells translate INSL4 mRNAs into immunoreactive pro-EPIL peptides comprising the B-, C- and A-chains (see Figure 1). Initially, pro-EPIL peptide was detected in amniotic fluid and maternal serum during normal pregnancy.

The amino-acid and mRNA sequence of the human Early placental insulin-like protein (INSL4 or EPIL, see GenPep accession number NP_002186) is:
Complete pro-EPIL amino acids sequence (SEQ ID NO: 1)

| | | | | | |
|---|---|---|---|---|---|
| 1 | MASLFRSYLP | AIWLLLSQLL | RESLAAELRG | CGPRFGKHLL | SYCPMPEKTF |
| 51 | TTTPGGWLLE | SGRPKEMVST | SNNKDGQALG | TTSEFIPNLS | PELKKPLSEG |
| 101 | QPSLKKIILS | RKKRSGRHRF | DPFCCEVICD | DGTSVKLCT | |

aal-22: signal peptide (SEQ ID NO: 2); aa23-52: "B chain" (SEQ ID NO: 3); aa59-108: "C chain (SEQ ID NO: 4); and aa115-139: "A chain" (SEQ ID NO: 5).
Insulin-like 4(placenta) (INSL4), mRNA (see Genbank accession number NM_002195) (SEQ ID NO: 6)

| | |
|---|---|
| 1 | agtctggagc ccagaaggga cacaccagca cagtctggta ggctacagca gcaagtctct |
| 61 | aaagaaaggc tgagaacacc cagaacagga gagttcaggt ccaggatggc cagcctgttc |
| 121 | cggtcctatc tgccagcaat ctggctgctg ctgagccaac tccttagaga aagcctagca |
| 181 | gcagagctga ggggatgtgg tccccgattt ggaaaacact tgctgtcata ttgccccatg |
| 241 | cctgagaaga cattcaccac caccccagga gggtggctgc tggaatctgg acgtcccaaa |
| 301 | gaaatggtgt caacctccaa caacaaagat ggacaagcct taggtacgac atcagaattc |
| 361 | attcctaatt tgtcaccaga gctgaagaaa ccactgtctg aagggcagcc atcattgaag |
| 421 | aaaataatac tttcccgcaa aaagagaagt ggacgtcaca gatttgatcc attctgttgt |
| 481 | gaagtaattt gtgacgatgg aacttcagtt aaattatgta catagtagag taatcatgga |
| 541 | ctggacatct catccattct catatgtatt ctcaatgaca aattcactga tgcccaatta |
| 601 | aatgattgct gtttaaa |

nt106-522:pro-EPIL coding sequence (SEQ ID NO: 7); nt106-17signal peptide (SEQ ID NO: 8); nt172-261 "B chain" (SEQ ID NO: 9); nt280-429 "C chain (SEQ ID NO: 10); and nt448-522: "A chain" (SEQ ID NO: 11).

INSL4 gene encodes a precursor that undergoes post-translational cleavage to produce 3 polypeptide chains, A-C, that form tertiary structures composed of either all three chains, or just the A and B chains (see European patent document Bellet et al., published under the number EP 2 281 195 B 1 on February 22, 2012).

It shall be understood that the term "specific peptide fragment" designates in particular a fragment of an amino acid sequence of a polypeptide having at least one of the functional characteristics or properties of the complete polypeptide, notably in that it is capable of being recognized by a specific antibody and/or that the expression level of such a specific peptide fragment is correlated to expression level of the complete or partial pro-EPIL expressed.

It is understood that the term "specific peptide fragment" designates particularly a polypeptide including a minimum of 9 amino acids, preferably 10, 11 or 12 amino acids, and most preferably 15, 20 or 25 amino acids of the sequence SEQ ID No: 1, preferably this fragment contains a fragment of at least the chain A, B or C of the human pro-EPIL.

Specific anti-pro-EPIL monoclonal or polyclonal antibodies are available to the skilled man. An isolated pro-EPIL, or a specific fragment thereof, can be used as an immunogen to generate antibodies that bind such protein using standard techniques for polyclonal and monoclonal antibody preparation. It may be also possible to use any fragment of these protein which contains at least one antigenic determinant may be used to generate these specific antibodies.

A protein immunogen typically is used to prepare antibodies by immunizing a suitable subject, (e.g., rabbit, goat, mouse or other mammal) with the immunogen. An appropriate immunogenic preparation can contain said pro-EPIL polypeptide, or fragment thereof, and further can include an adjuvant, such as Freund's complete or incomplete adjuvant, or similar immuno-stimulatory agent.

Monoclonal antibody (mAb) directed specifically (capable of binding) to the EPIL C-chain 98-108 region, such EPIL15 mAB (preferably as capture antibody) and/or mAb directed specifically (capable of binding) to the EPIL A-chain 125-137, such a EPIL02 (preferably as tracer) ate more preferred.

The alpha subunit of Human chorionic gonadotropin (HCG), which is a member of the glycoprotein hormone family, is encoded by one gene on chromosome 12q21.1-23. The HCGβ subunit however, is encoded by six non-allelic genes (CGB genes). The sequencing of the human genome offers a novel opportunity to check the sequences and organization of these genes clustered on chromosome 19q13.3 and named CGB1 or β1, CGB2 or β2, CGB3 or β3, CGB5 or β5 (SEQ ID No: 12 and SEQ ID No. 13, CGB7 or β7 and CGB8 or β8 [54-56].Genes β1 and β2 are considered pseudogenes that are not expressed whereas the remaining four genes encode the same protein, with the exception of β7 gene which encodes an alanine at position 117 as opposed to an aspartic acid in the other three genes [56-58]. On the basis of the amino acid residues displayed at position 117, genes encoding the HCGβ subunit were classified as type I genes if they encoded an alanine (β7) or as type II genes if they encoded an aspartic acid (β3, β5, β8).

In another particular aspect, the present invention is directed to a method for diagnosing pathology associated to epigenetic alteration and, optionally, to global hypomethylation, of cell in a patient, said method comprises the step of:
a) specifically detecting or quantifying the presence of the pro-EPIL peptide and/or of HCGβ type II subunit in a serum or plasma sample from said patient susceptible of containing pro-EPIL and/or HCGβ subunits type II, by a method which implements the use of a polyclonal or a monoclonal antibody (mAb) specifically directed to said pro-EPIL peptide, or to a specific fragment thereof, and/or pro-EPIL peptide and/or the use of a polyclonal antibody or mAb specifically directed to said HCGβ type II subunit, or to a specific fragment thereof.
   In another particular aspect, the present invention is directed to a method for detecting a predisposition to, or the incidence of, placental-related pathology or cancer pathology in a patient, said method comprises the step of:
   a) specifically detecting or quantifying the presence of the pro-EPIL peptide and/or of HCGβ type II subunit peptide in a serum or plasma sample from said patient susceptible of containing pro-EPIL and/or HCGβ subunits type II peptide, by a method which implements the use of a polyclonal antibody or a mAb specifically directed to said pro-EPIL peptide, or to a specific fragment thereof, and/or the use of a polyclonal antibody or a mAb specifically directed to said HCGβ type II subunit, or to a specific fragment thereof.

In a preferred embodiment, said antibody specifically directed to said pro-EPIL peptide or to said HCGβ type II subunit peptide is selected from the group consisting of:
a) polyclonal, monoclonal chimeric or humanized antibodies able to selectively bind, or which selectively bind to an epitope-containing a pro-EPIL polypeptide comprising a contiguous span of at least 9 to 10 amino acids of a pro-EPIL fragment, particularly of a fragment of at least the chain A, B or C of the human pro-EPIL; or
b) polyclonal, monoclonal chimeric or humanized antibodies able to selectively bind HCGβ type II subunit, or a specific fragment thereof, preferably able to selectively bind a discontinuous epitope that comprises region 1-7 with a lysine and a proline residue at position 2 and 4 respectively and region 82-92 of HCGβ, or an anti HCGβ type II-mAb specific binding fragment thereof.

In a more preferred embodiment, this antibody specifically directed to said HCGβ type II subunit peptide is selected from the group consisting of:
- a monoclonal antibody (mAb) specifically directed to a discontinuous epitope that comprises region 1-7 with a lysine and a proline residue at position 2 and 4 respectively (SEQ ID No. 14) and region 82-92 of HCGβ,
- a mAb specifically directed to a discontinuous epitope that comprises region 1-7 with a lysine and a proline residue at position 2 and 4 respectively and region 82-92 of HCGβ, wherein this antibody is produced by an hybridoma obtained from a mouse which has been prior immunized with an antigen comprising at least the fragments 1-7 with a lysine and a proline residue at position 2 and 4 respectively and 82-92 of HCGβ, said hybridoma being selected based on the capability of its secreted mAb to specifically recognizing the fragments 1-7 with a lysine and a proline residue at position 2 and 4 of the HCGβ type II;
- the mAb FBT-11-II produced by the hybridoma deposited with the CNCM (Collection Nationale de Cultures de Microorganismes, Institut Pasteur, 25 rue du Docteur Roux, F-75724 PARIS Cedex 15) on March 9, 2010 under the number I-4281;
- the mAb FBT-11 produced by the hybridoma deposited with the CNCM on October 3, 1985 under the number I-489;
- a recombinant mAb having a sequence comprising at least the 6 CDRs (Complementary Determining Region) of the mAb FBT-11-II produced by the hybridoma deposited under the number I-4281or at least the 6 CDRs of the the mAb FBT-11 produced by the hybridoma deposited under the number I-489; and
- a HCGβ type II-binding fragment thereof.

In the method of the present invention, the determination of the presence or the level of said biomarker in a serum or plasma sample of said patient is carried out preferably by immunoassay, more preferably by ELISA immunoassay or radioimmunoassay in blood serum or plasma.

When the presence or the increase of HCGβ subunits type II biomarker is desired to be determined in a serum or plasma sample, in a preferred embodiment of the the present invention this method comprises the steps of:
a) contacting the biological sample from the patient to be tested or from the control with an anti-HCGβ subunits type II antibody selected from the group as described above or a HCGβ type II-binding fragment thereof,
   preferably under conditions permitting the binding of said antibody to the HCGβ type II subunits present in said biological sample; and
b) measuring the amount of the complex formed between said antibody bound to the HCGβ type II subunits so as to thereby determine the amount of HCGβ type II in the sample.

In a preferred embodiment of the present invention, said method for specifically detecting or quantifying the presence of HCGβ type II subunits in a biological sample comprises the steps of:
a) contacting the biological sample from the subject with a capture antibody capable of binding HCGβ type I and type II under conditions permitting the formation of a complex between the antibody and any HCGβ present in the sample;
b) contacting the complex formed with a second antibody (tracer antibody) which is a specific anti-HCGβ subunits type II antibody selected from the group as described above or a HCGβ type II-binding fragment thereof,
   preferably under conditions permitting the binding of said antibody to the HCGβ type II subunits present in said biological sample; and
c) measuring the amount of the second antibody bound to the complex formed so as to thereby determine the amount of HCGβ type II in the sample.

According to the present invention and in a more preferred embodiment,
- in step a), the capture antibody is bound to a solid support and the step comprises the removing of any unbound sample from the solid support; and
- in step b), the solid support is contacted with the second antibody.

In a more preferred embodiment, the first antibodies used (capture antibody) are mAbs directed to the carboxyl terminal portion of HCGβ, preferably directed to an epitope comprising at least 6 amino acid residues, preferably at least 10, 12, 15 or 20 residues between the fragment AA118-147 of the HCGβ type I or II, and/or preferably, also directed to an epitope comprising at least 6 amino acid residues, preferably at least 10, 12, or 15 residues between the fragment AA95-116 of the HCGβ type I or II preferably the monoclonal antibodies (mAbs) named FB09 or FB12 which were obtained as referenced in the Examples.

The antibody, named FBT10, secreted by the hybridoma deposited under the number 1-488 with the CNCM on October 3, 1985 can be also used as capture antibody in the method of the present invention.

In a more preferred embodiment, the antibodies anti-pro-EPIL or anti-HCGβ type II used, particularly can be labelled antibody.

"Labelled antibody" as used herein includes antibodies that are labeled by a detectable means and includes enzymatically, radioactively, fluorescently, chemiluminescently, bioluminescently, biotin or magnetic bead labeled antibodies by any of the many different methods known to those skilled in this art.

One of the ways in which an antibody can be detectably labelled is by linking the same antibody to an enzyme. This enzyme, in turn, when later exposed to its substrate, will react with the substrate in such a manner as to produce a chemical moiety which can be detected, for example, by spectrophotometric, fluorometric or by visual means. Enzymes which can be used to detectably label pro-EPIL or HCGβ type II-specific antibody include, but are not limited to, malate dehydrogenase, staphylococcal nuclease, delta-V-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholine sterase.

Detection or quantification may be accomplished using any of a variety of immunoassays. For example, by radioactively labelling an antibody, it is possible to detect the antibody through the use of radioimmune assays. A description of a radioimmune assay (RIA) may be found in Laboratory Techniques and Biochemistry in Molecular Biology, by Work T. S. et al., North Holland Publishing Company, NY (1978), with particular reference to the chapter entitled "An Introduction to Radioimmune Assay and Related Techniques" by Chard T. The radioactive isotope can be detected by such means as the use of a gamma counter or a scintillation counter or by audioradiography. Isotopes which are particularly useful for the purpose of the present invention are: ³H, ¹³¹I, ³⁵S, ¹⁴C, and preferably ¹²⁵I.

It is also possible to label an antibody with a fluorescent compound. When the fluorescently labelled antibody is exposed to light of the proper wave length, its presence can then be detected due to fluorescence. Among the most commonly used fluorescent labelling compounds are fluorescein isothiocyanate, rhodamine, phycoerytherin, phycocyanin, allophycocyanin, ophthaldehyde and fluorescamine.

An antibody can also be detectably labelled using fluorescence emitting metals such as ¹⁵²Eu, or others of the lanthanide series. These metals can be attached to the antibody using such metal chelating groups as diethylenetriaminepentaacetic acid (DTPA) or ethylenediaminetetraacetic acid (EDTA).

An antibody can also be detectably labelled by coupling it to a chemiluminescent compound. The presence of the chemiluminescent-tagged antibody is then determined by detecting the presence of luminescence that arises during the course of a chemical reaction. Examples of particularly useful chemiluminescent labelling compounds are luminol, luciferin, isoluminol, theromatic acridinium ester, imidazole, acridinium salt and oxalate ester.

Likewise, a bioluminescent compound may be used to label an antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labelling are luciferin, luciferase and aequorin.

In the detection or quantification assays implemented in the method of the invention, the amount of binding of the antibody to the biological sample can be determined by the intensity of the signal emitted by the labelled antibody and/or by the number cells in the biological sample bound to the labelled antibody.

The detection or the level of the selected serum biomarker of epigenetic alterations, particularly of global hypomethylation, such as pro-EPIL or HCGβ type II biomarker, in a biological sample may be determined by a radioimmunoassay, an immunoradiometric assay, and/or an enzyme immunoassay.

"Radioimmunoassay" is a technique for detecting and measuring the concentration of an antigen using a labelled (i.e. radioactively labelled) form of the antigen (HCGβ type II). The concentration of HCGβ type II in a biological sample is measured by having the antigen in the sample compete with a labelled (i.e. radioactively) antigen for binding to an antibody to the antigen. To ensure competitive binding between the labelled antigen and the unlabeled antigen, the labelled antigen is present in a sufficient concentration to saturate the binding sites of the antibody. The higher the concentration of antigen in the sample, the lower the concentration of labelled antigen that will bind to the antibody.

In a radioimmunoassay, to determine the concentration of labelled antigen bound to an antibody, the antigen-antibody complex must be separated from the free antigen. One method for separating the antigen-antibody complex from the free antigen is by precipitating the antigen-antibody complex with an anti-isotype antiserum. Another method for separating the antigen-antibody complex from the free antigen is by precipitating the antigen-antibody complex with formalin-killed S. aureus. Yet another method for separating the antigen-antibody complex from the free antigen is by performing a "solid-phase radioimmunoassay" where the antibody is linked (i.e. covalently) to Sepharose beads, polystyrene wells, polyvinylchloride wells, or microtiter wells. By comparing the concentration of labelled antigen bound to antibody to a standard curve based on samples having a known concentration of antigen, the concentration of antigen in the biological sample can be determined.

An "Immunoradiometric assay" (IRMA) is an immunoassay in which the antibody reagent is radioactively labeled. An IRMA requires the production of a multivalent antigen conjugate by techniques such as conjugation to a protein e.g., rabbit serum albumin (RSA). The multivalent antigen conjugate must have at least 2 epitopes per molecule and these epitopes must be located at a sufficient distance to allow binding by at least two antibodies to the antigen. For example, in an IRMA the multivalent antigen conjugate can be attached to a solid surface such as a plastic sphere.

Unlabelled "sample" antigen and antibody to antigen which is radioactively labelled are added to a test tube containing the multivalent antigen conjugate coated sphere. The antigen in the sample competes with the multivalent antigen conjugate for antigen antibody binding sites. After an appropriate incubation period, the unbound reactants are removed by washing and the amount of radioactivity on the solid phase is determined. The amount of bound radioactive antibody is inversely proportional to the concentration of antigen in the sample.

The most common enzyme immunoassay is the "Enzyme-Linked Immunosorbent Assay (ELISA)". The "Enzyme-Linked Immunosorbent Assay (ELISA)" is a technique for detecting and measuring the concentration of an antigen using a labelled (i.e. enzyme linked) form of the antibody.

In a "sandwich ELISA", an antibody (i.e. anti-pro-EPIL or anti- HCGβ) is linked to a solid phase (i.e. a microtiter plate) and exposed to a biological sample containing antigen (i.e. pro-EPIL or HCGβ type II). The solid phase is then washed to remove unbound antigen. A labelled antibody (i.e. anti-pro-EPIL or anti- HCGβ type II enzyme linked) is then bound to the bound-antigen (if present) forming an antibody-antigen-antibody sandwich. Examples of enzymes that can be linked to the antibody are alkaline phosphatase, horseradish peroxidase, luciferase, urease, and 3-galactosidase. The enzyme linked antibody reacts with a substrate to generate a colored reaction product that can be assayed for.

In a "competitive ELISA", antibody is incubated with a sample containing antigen (i.e. HCGβ type II). The antigen-antibody mixture is then contacted with an antigen-coated solid phase (i.e. a microtiter plate). The more antigen present in the sample, the less free antibody that will be available to bind to the solid phase. A labelled (i.e. enzyme linked) secondary antibody is then added to the solid phase to determine the amount of primary antibody bound to the solid phase.

In another aspect, the present invention is directed to a kit for diagnosing pathology associated to epigenetic alteration, particularly to global hypomethylation, of cell in a patient, said kit comprising:
a) an antibody directed specifically against the pro-EPIL peptide, or specific fragment thereof; or/and
b) an antibody directed specifically against the HCGβ-type II subunit peptide, or specific fragment thereof.

In a preferred embodiment, said pathology associated to epigenetic alterations is a cancer or a placental-related pathology. Preferrably, said pathology associated to epigenetic alterations is a placental-related pathology is selected from the group consisting of small for gestational age (SGA), intrauterine growth retardation (IUGR) and pre-eclampsia and/or said cancer is selected from tumorigenic or metastatic cancer, more preferably selected from breast, bladder, colorectal or lung cancer.

In another aspect, the present invention is directed to a kit for detecting a predisposition to, or the incidence of, placental-related pathology or cancer pathology in a patient, said kit comprising:
a) an antibody directed specifically against the pro-EPIL peptide, or a binding fragment thereof, optionally labelled; or/and
b) an antibody directed specifically against the HCGβ-type II subunit peptide, or a binding fragment thereof, optionally labelled.

In a preferred embodiment, said antibody specifically directed to said pro-EPIL peptide or to said HCGβ type II subunit peptide contained in the kit of the present invention is selected from the group consisting of:
a) polyclonal, monoclonal chimeric or humanized antibodies able to selectively bind, or which selectively bind to an epitope-containing a pro-EPIL polypeptide comprising a contiguous span of at least 9 to 10 amino acids of a pro-EPIL fragment, particularly of a fragment of at least the chain A, B or C of the human pro-EPIL; or
b) polyclonal, monoclonal chimeric or humanized antibodies able to selectively bind HCGβ type II subunit, or a specific fragment thereof, preferably able to selectively bind a discontinuous epitope that comprises region 1-7 with a lysine and a proline residue at position 2 and 4 respectively and region 82-92 of HCGβ, or an anti HCGβ type II-mAb specific binding fragment thereof.

In a more preferred embodiment, this antibody specifically directed to said HCGβ type II subunit peptide is selected from the group consisting of:
- a monoclonal antibody (mAb) specifically directed to a discontinuous epitope that comprises region 1-7 with a lysine and a proline residue at position 2 and 4 respectively and region 82-92 of HCGβ,
- a mAb specifically directed to a discontinuous epitope that comprises region 1-7 with a lysine and a proline residue at position 2 and 4 respectively and region 82-92 of HCGβ, wherein this antibody is produced by an hybridoma obtained from a mouse which has been prior immunized with an antigen comprising at least the fragments 1-7 with a lysine and a proline residue at position 2 and 4 respectively and 82-92 of HCGβ, said hybridoma being selected based on the capability of its secreted mAb to specifically recognizing the fragments 1-7 with a lysine and a proline residue at position 2 and 4 of the HCGβ type II;
- the mAb FBT-11-II produced by the hybridoma deposited with the CNCM (Collection Nationale de Cultures de Microorganismes, Institut Pasteur, 25 rue du Docteur Roux, F-75724 PARIS Cedex 15) on March 9, 2010 under the number I-4281;
- the mAb FBT-11 produced by the hybridoma deposited with the CNCM on October 3, 1985 under the number I-489;
- a recombinant mAb having a sequence comprising at least the 6 CDRs (Complementary Determining Region) of the mAb FBT-11-II produced by the hybridoma deposited under the number I-4281or at least the 6 CDRs of the the mAb FBT-11 produced by the hybridoma deposited under the number I-489; and
- a HCGβ type II-binding fragment thereof.

The following examples and figures are given for the purpose of illustrating various embodiments of the invention and are not meant to limit the present invention in any fashion.

### LEGENDS OF THE FIGURES

**Figure 1****:** Schematic representation of the primary structure of pro-EPIL peptide and localization of antibody binding sites recognized by monoclonal antibodies EPIL15 and EPIL02. Amino acid residues are indicated by one-letter code.

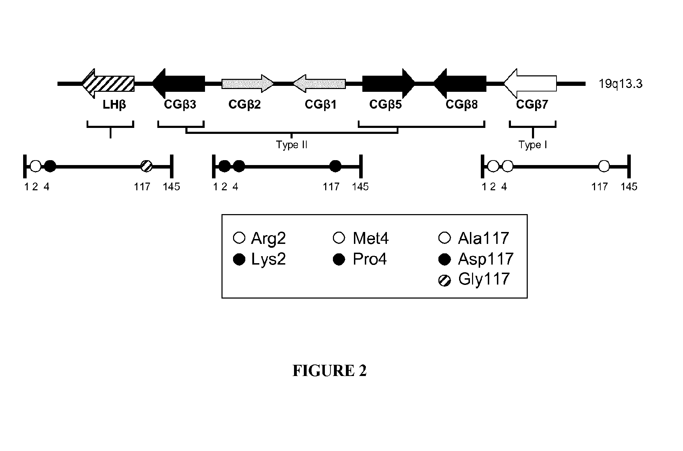

**Figure 2****:** Organization of the CGβ/LHβ gene cluster and amino acid sequences of expressed genes. Only genes CGβ3, CGβ5, CGβ7 and CGβ8 code for the hCGβ subunit.
Type I genes code for a mature protein with an arginine residue at amino acid 2, a methionine residue at amino acid 4 (see SEQ ID No. 15) and an alanine residue at amino acid 117. Type II genes encode a mature protein with a lysine residue at amino acid 2, a proline residue at amino acid 4 and an aspartic acid at amino acid 117.

### EXAMPLE 1: MATERIALS AND METHODS

(see International patent application Bellet et al., filed under N° EP2011/053676 on March 11, 2011)

### Monoclonal antibodies

Monoclonal antibody (mAb) EPIL15 is directed to the EPIL C-chain 98-108 region. This antibody serves as capture antibody on a solid phase support, while biotinylated mAb EPIL02 directed to the EPIL A-chain 125-137 region is used as tracer. The standard curve was constructed with a peptide spanning the 76-139 portion of pro-EPIL used at increasing concentrations ranging from 0.7 ng/mL to 100 ng/mL. Linearity in a range of 0.7 ng/mL to 100 ng/mL was consistently shown in between and within-run assays. The sensitivity of this ELISA is 1 ng/mL (see European patent document, Bellet et al., published under the number EP 2 281 195 B1 on February 22, 2012).

Monoclonal antibody (mAbs) FBT-11-II is an IgG1-Kappa produced by the hybridoma deposited pursuant to and in satisfaction of, the requirements of the Budapest Treaty on the International Recognition of the Deposit of Microorganisms for the Purposes of Patent Procedure with the CNCM (Collection Nationale de Cultures de Microorganismes, Institut Pasteur, 25 rue du Docteur Roux, F-75724 PARIS Cédex 15) on March 9, 2010 under the number I-4281.

The hybridoma I-4281 which secretes the FBT-11-II antibody results from successive subcloning cycles of the the hybridoma I-489 also deposited pursuant to and in satisfaction of, the requirements of the Budapest Treaty on the International Recognition of the Deposit of Microorganisms for the Purposes of Patent Procedure with the CNCM on October 3, 1985.

Monoclonal antibodies (mAbs) FB09, FB12 and FBT11-II were obtained as previously described ((see International patent application filed under N° EP2011/053676 on 11 March 2011 for references).

MAbs FB09 and FB12, elicited against a synthetic peptide analogous to the COOH 109-145 terminal portion (CTP) of HGGβ, are directed against the 134-139 and 110-116 regions, respectively (23). These mAbs are specific for either HCG or its HCGβ subunit and do not bind to LH or its LHβ subunit. MAb FBT11 and FBT-11-II secreted elicited against purified HCGβ subunit (CR 129), are directed to a discontinuous epitope that comprises region 1-7 with a lysine and a proline residue at position 2 and 4 respectively and region 82-92 of HCGβ. FBT-11 and FBT-11-II are specific for the HCGβ subunit and do not bind to HCG, LH or its LHβ subunit (20).

### Two-site ELISA (measure of the HCGβ type II level)

Maxisorp nunc plates (Thermo Fisher Scientific, Brebières, France) were coated with 0.25 µg of monoclonal antibody FB09 and/or 0.25 µg of monoclonal antibody FB12 in 0.1M phosphate buffer pH 7.4, blocked with 1% bovine serum albumin in PBS and incubated with the HCGβ standards (ELSA-FBHCG from CIS Bio International, France; 1 ng CIS= 1 mIU 1^{st} IRP WHO 75/551) for 1h at 37°C. Bound HCGβ was detected with monoclonal antibody FBT11-II coupled with biotin for 1h at 37°C (Biotin Labeling Kit -NH₂ from Interchim, Montluçon, France). The plate was then incubated with Immunopure streptavidin Horseradish peroxydase conjugated (Pierce, Thermo Fisher Scientific, Brebières, France) for 10 min at room temperature (RT). TMB from Pierce was used as the substrate and the absorbance was read at 450 nm. Experiments were done in duplicate. The standard curve was constructed with the HCGβ standards used at increasing concentrations ranging from 0.21-47 ng/ml. Linearity was consistently shown in between run assays.

It is known that FBT11-II specifically recognizes HCGβ encoded by type II genes at the cellular level and FBT11-II recognizes HCGβ encoded by type II genes and produced by trophoblasts during the course of gestation.

It has already be concluded that FBT11 and FBT11-II are directed against a highly specific and discontinuous epitope that comprises region 1-7 with a lysine and a proline residue at position 2 and 4 respectively and region 82-92 of HCGβ (see International patent application filed under N° EP2011/053676 on March 11, 2011)

### ELISA specific for HCGβ encoded by type II genes

It has been also demonstrated in that cited patent document filed under N° EP2011/053676 on March 11, 2011, that enhanced binding of indicator antibody to HCG was obtained using anti-CTP antibodies FB09 and FB12 as capture antibodies. This assay could be useful to determine the presence or absence and the level of HCGβ encoded by type II genes in biological fluids such as serum or plasma.

### EXAMPLE II: Presence in the serum of cancer patients of pro-EPIL that is expressed as a consequence of epigenetic alterations in both placental and cancer cells, Pro-EPIL peptide as surrogate biomarker of both epigenetic alterations, particularly of global hypomethylation.

The first biomarker, pro-EPIL is a peptide encoded by INSL4 gene [59, 60]. In normal tissues, INSL4 is expressed strongly only in placenta. Much lower expression (∼ 10,000 times lower than the placenta) is detected in thymus, testis breast, stomach and uterus [61]. The other normal tissues contained very little or no detectable INSL4 mRNA. The placenta specific expression of INSL4 is mediated by the 3' LTR of the HERV element inserted in the INSL4 promoter. This placenta specific expression via HERV element integration could contribute to the aggressive growth phenotype of normal placenta. Moreover, INSL4 gene is also expressed by trophoblastic and nontrophoblastic cancer cells and may play a role in the invasive properties of malignant cells [62, 63]. Interestingly, amongst genes which have a placenta specific expression due to hypomethylation of the retrotransposons that regulate their expression, including KCNH5, ERVWE1, EDNRB, PTN or MIDI, the INSL4 gene has the advantage of being transcribed and translated into a well-identified peptide designated as pro-EPIL. This peptide can be detected in biological fluids using a two-site immunoassay based on monoclonal antibodies and is present in detectable amount in the serum [64, 65].

### EXAMPLE III: Presence in the serum of cancer patients of free hCGβ type II that is expressed as a consequence of epigenetic alterations in both placental and cancer cells, free hCGβ type II as surrogate biomarker of both epigenetic alterations and global hypomethylation.

This second biomarker, human chorionic gonadotropin beta subunit type II (free hCGβ type II), is a protein encoded by type II CGB genes CGB3, CGB5 and CGB8.The expression of these type II CGB genes is upregulated in both trophoblastic and neoplastic cells while type I CGB gene CGB7 which encodes free hCGβ type I is expressed in many normal cells [66]. In placenta, it has been shown that the entire CGB locus appears to be very undermethylated, leading to the expression of type II CGB genes at very high levels. Similarly, the most likely explanation for the transcription of type II CGB genes in neoplastic cells of differing histological origin might be that demethylation of critical sites plays a major role in the transcriptional activation of type II CGB genes. The free hCGβ type II is detected in the serum of patients with trophoblastic and non-trophoblastic cancers of various histological origin [67]. Until now, it has been difficult to prove that a sensitive immunoassay assays would be capable of specifically measuring the presence of free hCGβ type II in the serum without detecting free hCGβ type I. Recently, it was found that a monoclonal antibody was capable of specifically recognizing the free hCGβ type II thanks to its binding to an antigenic sites that comprises two amino acid residues only present on free hCGβ type II and absent from free hCGβ type I. Thus, the hypomethylated state of type II CGB genes in both placenta and cancer and the possibility of specifically detecting the protein products of these genes in serum lead to the selection of free hCGβ type II as a protein biomarker that can complement pro-EPIL as surrogate biomarkers of epigenetic alterations and global hypomethylation, a specific feature of cancer cells.

### EXAMPLE IV: Results

The levels of pro-EPIL and free hCGβ type II in the serum of patients with lung, bladder or colorectal cancers using specific two-site immunoassays based on monoclonal antibodies. The first immunoassay is based on monoclonal antibody (mAb) EPIL15 as capture antibody and mAb EPIL 02 as tracer for measuring pro-EPIL while the sandwich immunoassay utilized for measuring free hCGβ type II is based on mAb FBT11-II. The assay for pro-EPIL has a functional sensitivity of 1 ng/ml while the functional sensitivity of the assay for free hCGβ type II is 0.1 ng/ml. Serum levels of pro-EPIL were higher than 1 ng/ml in 2 out of 100 apparently healthy women and in 3 out of 100 apparently healthy men.

Serum levels of free hCGβ type II were consistently lower than 0.1 ng/ml in our series of 100 apparently healthy women and 100 apparently healthy men. In a series of 129 patients with lung cancer, 28 (21.7%) patients had increased serum levels of pro-EPIL (> 1 ng/ml), 35 (27%) patients had increased serum levels of hCGβ type II (>0.1ng/ml). Indeed, 54 (41.8%) patients with lung cancers display increased serum levels of either pro-EPIL, free hCGβ type II or both. In a series of 132 patients with colorectal cancer, 9 (6.8%) patients had increased serum levels of pro-EPIL (> 1 ng/ml), 14 (10.6%) patients had increased serum levels of HCGβ type II (>0.1ng/ml). Indeed, 23 (17.4%) patients with colorectal cancers display increased serum levels of either pro-EPIL or free hCGβ type II. In a series of 10 patients with bladder cancer, 1 (10%) had increased serum levels of pro-EPIL (> 1 ng/ml), 3 (30%) had increased serum levels of hCGβ type II (>0.1ng/ml). Indeed, 4 (40%) patients with bladder cancers display increased serum levels of either pro-EPIL or free hCGβ type II.

Thus, in addition to cancer diseases, Pro-EPIL and hCGβ type II can also be used as surrogate biomarkers of epigenetic alterations associated with various pathologies of pregnancy including of intrauterine growth retardation, pre-eclampsia or chromosomally abnormal pregnancies. As a first example, it is striking that the transcription levels of both INSL4 and type II CGB genes are increased in chromosomally abnormal pregnancies [68, 69]. Moreover, free hCGβ is already a serum biomarker widely used for the screening of Down's syndrome [69] and it is highly likely that hCGβ type II might constitute a better biomarker of these chromosomally abnormal pregnancies, alone, in combination with pro-EPIL or in combination with other serum markers of epigenetic alterations and global hypomethylation which can be selected by the method of the present invention.

### REFERENCES

1. Nelissen EC, van Montfoort AP, Dumoulin JC, Evers JL: Epigenetics and the placenta. Human reproduction update 2011, 17(3):397-417.
2. Reik W: Stability and flexibility of epigenetic gene regulation in mammalian development. Nature 2007, 447(7143):425-432.
3. Feinberg AP, Tycko B: The history of cancer epigenetics. Nature reviews Cancer 2004, 4(2):143-153.
4. Qiu J: Epigenetics: unfinished symphony. Nature 2006, 441(7090):143-145.
5. Gama-Sosa MA, Midgett RM, Slagel VA, Githens S, Kuo KC, Gehrke CW, Ehrlich M: Tissue-specific differences in DNA methylation in various mammals. Biochimica et biophysica acta 1983, 740(2):212-219.
6. Ehrlich M: DNA methylation in cancer: too much, but also too little. Oncogene 2002, 21(35):5400-5413.
7. Ehrlich M, Gama-Sosa MA, Huang LH, Midgett RM, Kuo KC, McCune RA, Gehrke C: Amount and distribution of 5-methylcytosine in human DNA from different types of tissues of cells. Nucleic acids research 1982, 10(8):2709-2721.
8. Feinberg AP, Vogelstein B: Hypomethylation distinguishes genes of some human cancers from their normal counterparts. Nature 1983, 301(5895):89-92.
9. Gama-Sosa MA, Slagel VA, Trewyn RW, Oxenhandler R, Kuo KC, Gehrke CW, Ehrlich M: The 5-methylcytosine content of DNA from human tumors. Nucleic acids research 1983, 11(19):6883-6894.
10. Adorjan P, Distler J, Lipscher E, Model F, Muller J, Pelet C, Braun A, Florl AR, Gutig D, Grabs G et al: Tumour class prediction and discovery by microarray-based DNA methylation analysis. Nucleic acids research 2002, 30(5):e21.
11. Iacobuzio-Donahue CA, Maitra A, Olsen M, Lowe AW, van Heek NT, Rosty C, Walter K, Sato N, Parker A, Ashfaq R et al: Exploration of global gene expression patterns in pancreatic adenocarcinoma using cDNA microarrays. The American journal of pathology 2003, 162(4):1151-1162.
12. Oshimo Y, Nakayama H, Ito R, Kitadai Y, Yoshida K, Chayama K, Yasui W: Promoter methylation of cyclin D2 gene in gastric carcinoma. International journal of oncology 2003, 23(6):1663-1670.
13. Akiyama Y, Maesawa C, Ogasawara S, Terashima M, Masuda T: Cell-type-specific repression of the maspin gene is disrupted frequently by demethylation at the promoter region in gastric intestinal metaplasia and cancer cells. The American journal of pathology 2003, 163(5):1911-1919.
14. Cho M, Uemura H, Kim SC, Kawada Y, Yoshida K, Hirao Y, Konishi N, Saga S, Yoshikawa K: Hypomethylation of the MN/CA9 promoter and upregulated MN/CA9 expression in human renal cell carcinoma. Br J Cancer 2001, 85(4):563-567.
15. Nakamura N, Takenaga K: Hypomethylation of the metastasis-associated S100A4 gene correlates with gene activation in human colon adenocarcinoma cell lines. Clinical & experimental metastasis 1998, 16(5):471-479.
16. Badal V, Chuang LS, Tan EH, Badal S, Villa LL, Wheeler CM, Li BF, Bernard HU: CpG methylation of human papillomavirus type 16 DNA in cervical cancer cell lines and in clinical specimens: genomic hypomethylation correlates with carcinogenic progression. Journal of virology 2003, 77(11):6227-6234.
17. de Capoa A, Musolino A, Della Rosa S, Caiafa P, Mariani L, Del Nonno F, Vocaturo A, Donnorso RP, Niveleau A, Grappelli C: DNA demethylation is directly related to tumour progression: evidence in normal, pre-malignant and malignant cells from uterine cervix samples. Oncology reports 2003, 10(3):545-549.
18. Sato N, Maitra A, Fukushima N, van Heek NT, Matsubayashi H, Iacobuzio-Donahue CA, Rosty C, Goggins M: Frequent hypomethylation of multiple genes overexpressed in pancreatic ductal adenocarcinoma. Cancer Res 2003, 63(14):4158-4166.
19. Piyathilake CJ, Henao O, Frost AR, Macaluso M, Bell WC, Johanning GL, Heimburger DC, Niveleau A, Grizzle WE: Race- and age-dependent alterations in global methylation of DNA in squamous cell carcinoma of the lung (United States). Cancer causes & control: CCC 2003, 14(1):37-42.
20. Ehrlich M, Jiang G, Fiala E, Dome JS, Yu MC, Long TI, Youn B, Sohn OS, Widschwendter M, Tomlinson GE et al: Hypomethylation and hypermethylation of DNA in Wilms tumors. Oncogene 2002, 21(43):6694-6702.
21. Dante R, Dante-Paire J, Rigal D, Roizes G: Methylation patterns of long interspersed repeated DNA and alphoid repetitive DNA from human cell lines and tumors. Anticancer research 1992, 12(2):559-563.
22. Santourlidis S, Florl A, Ackermann R, Wirtz HC, Schulz WA: High frequency of alterations in DNA methylation in adenocarcinoma of the prostate. The Prostate 1999, 39(3):166-174.
23. Jurgens B, Schmitz-Drager BJ, Schulz WA: Hypomethylation of L1 LINE sequences prevailing in human urothelial carcinoma. Cancer Res 1996, 56(24):5698-5703.
24. Leib-Mosch C, Haltmeier M, Werner T, Geigl EM, Brack-Werner R, Francke U, Erfle V, Hehlmann R: Genomic distribution and transcription of solitary HERV-K LTRs. Genomics 1993, 18(2):261-269.
25. Herbst H, Sauter M, Mueller-Lantzsch N: Expression of human endogenous retrovirus K elements in germ cell and trophoblastic tumors. The American journal of pathology 1996, 149(5):1727-1735.
26. Wang-Johanning F, Frost AR, Johanning GL, Khazaeli MB, LoBuglio AF, Shaw DR, Strong TV: Expression of human endogenous retrovirus k envelope transcripts in human breast cancer. Clinical cancer research : an official journal of the American Association for Cancer Research 2001, 7(6):1553-1560.
27. Qu G, Dubeau L, Narayan A, Yu MC, Ehrlich M: Satellite DNA hypomethylation vs. overall genomic hypomethylation in ovarian epithelial tumors of different malignant potential. Mutation research 1999, 423(1-2):91-101.
28. Ribieras S, Song-Wang XG, Martin V, Lointier P, Frappart L, Dante R: Human breast and colon cancers exhibit alterations of DNA methylation patterns at several DNA segments on chromosomes 11p and 17p. Journal of cellular biochemistry 1994, 56(1):86-96.
29. Ferretti C, Bruni L, Dangles-Marie V, Pecking AP, Bellet D: Molecular circuits shared by placental and cancer cells, and their implications in the proliferative, invasive and migratory capacities of trophoblasts. Hum Reprod Update 2007, 13(2):121-141.
30. Koslowski M, Sahin U, Mitnacht-Kraus R, Seitz G, Huber C, Tureci O: A placenta-specific gene ectopically activated in many human cancers is essentially involved in malignant cell processes. Cancer Res 2007, 67(19):9528-9534.
31. Perry JK, Lins RJ, Lobie PE, Mitchell MD: Regulation of invasive growth: similar epigenetic mechanisms underpin tumour progression and implantation in human pregnancy. Clin Sci (Lond) 2010, 118(7):451-457.
32. Shen L, Fang J, Qiu D, Zhang T, Yang J, Chen S, Xiao S: Correlation between DNA methylation and pathological changes in human hepatocellular carcinoma. Hepato-gastroenterology 1998, 45(23):1753-1759.
33. Lin CH, Hsieh SY, Sheen IS, Lee WC, Chen TC, Shyu WC, Liaw YF: Genome-wide hypomethylation in hepatocellular carcinogenesis. Cancer Res 2001, 61(10):4238-4243.
34. Soares J, Pinto AE, Cunha CV, Andre S, Barao I, Sousa JM, Cravo M: Global DNA hypomethylation in breast carcinoma: correlation with prognostic factors and tumor progression. Cancer 1999, 85(1):112-118.
35. Feinberg AP, Gehrke CW, Kuo KC, Ehrlich M: Reduced genomic 5-methylcytosine content in human colonic neoplasia. Cancer Res 1988, 48(5):1159-1161.
36. Wild L, Flanagan JM: Genome-wide hypomethylation in cancer may be a passive consequence of transformation. Biochimica et biophysica acta 2010, 1806(1):50-57.
37. Gama-Sosa MA, Wang RY, Kuo KC, Gehrke CW, Ehrlich M: The 5-methylcytosine content of highly repeated sequences in human DNA. Nucleic acids research 1983, 11(10):3087-3095.
38. Kuo KC, McCune RA, Gehrke CW, Midgett R, Ehrlich M: Quantitative reversed-phase high performance liquid chromatographic determination of major and modified deoxyribonucleosides in DNA. Nucleic acids research 1980, 8(20):4763-4776.
39. Nephew KP, Balch C, Skalnik DG: Methyl group acceptance assay for the determination of global DNA methylation levels. Methods in molecular biology 2009, 507:35-41.
40. Frommer M, McDonald LE, Millar DS, Collis CM, Watt F, Grigg GW, Molloy PL, Paul CL: A genomic sequencing protocol that yields a positive display of 5-methylcytosine residues in individual DNA strands. Proc Natl Acad Sci U S A 1992, 89(5):1827-1831.
41. Trinh BN, Long TI, Laird PW: DNA methylation analysis by MethyLight technology. Methods 2001, 25(4):456-462.
42. Wong IH, Lo YM, Johnson PJ: Epigenetic tumor markers in plasma and serum: biology and applications to molecular diagnosis and disease monitoring. Annals of the New York Academy of Sciences 2001, 945:36-50.
43. Chen XQ, Stroun M, Magnenat JL, Nicod LP, Kurt AM, Lyautey J, Lederrey C, Anker P: Microsatellite alterations in plasma DNA of small cell lung cancer patients. Nature medicine 1996, 2(9):1033-1035.
44. Anker P, Lefort F, Vasioukhin V, Lyautey J, Lederrey C, Chen XQ, Stroun M, Mulcahy HE, Farthing MJ: K-ras mutations are found in DNA extracted from the plasma of patients with colorectal cancer. Gastroenterology 1997, 112(4):1114-1120.
45. Esteller M, Sanchez-Cespedes M, Rosell R, Sidransky D, Baylin SB, Herman JG: Detection of aberrant promoter hypermethylation of tumor suppressor genes in serum DNA from non-small cell lung cancer patients. Cancer Res 1999, 59(1):67-70.
46. Wong IH, Lo YM, Zhang J, Liew CT, Ng MH, Wong N, Lai PB, Lau WY, Hjelm NM, Johnson PJ: Detection of aberrant p16 methylation in the plasma and serum of liver cancer patients. Cancer Res 1999, 59(1):71-73.
47. Wong IH, Johnson PJ, Lai PB, Lau WY, Lo YM: Tumor-derived epigenetic changes in the plasma and serum of liver cancer patients. Implications for cancer detection and monitoring. Annals of the New York Academy of Sciences 2000, 906:102-105.
48. Issa JP, Gharibyan V, Cortes J, Jelinek J, Morris G, Verstovsek S, Talpaz M, Garcia-Manero G, Kantarjian HM: Phase II study of low-dose decitabine in patients with chronic myelogenous leukemia resistant to imatinib mesylate. Journal of clinical oncology : official journal of the American Society of Clinical Oncology 2005, 23(17):3948-3956.
49. Li S, Chiang TC, Richard-Davis G, Barrett JC, McLachlan JA: DNA hypomethylation and imbalanced expression of DNA methyltransferases (DNMT1, 3A, and 3B) in human uterine leiomyoma. Gynecologic oncology 2003, 90(1):123-130.
50. Santos F, Peters AH, Otte AP, Reik W, Dean W: Dynamic chromatin modifications characterise the first cell cycle in mouse embryos. Developmental biology 2005, 280(1):225-236.
51. Habib M, Fares F, Bourgeois CA, Bella C, Bernardino J, Hernandez-Blazquez F, de Capoa A, Niveleau A: DNA global hypomethylation in EBV-transformed interphase nuclei. Experimental cell research 1999, 249(1):46-53.
52. Down TA, Rakyan VK, Turner DJ, Flicek P, Li H, Kulesha E, Graf S, Johnson N, Herrero J, Tomazou EM et al: A Bayesian deconvolution strategy for immunoprecipitation-based DNA methylome analysis. Nature biotechnology 2008, 26(7):779-785.
53. Weber M, Davies JJ, Wittig D, Oakeley EJ, Haase M, Lam WL, Schubeler D: Chromosome-wide and promoter-specific analyses identify sites of differential DNA methylation in normal and transformed human cells. Nature genetics 2005, 37(8):853-862.
54. Boorstein WR, Vamvakopoulos NC, Fiddes JC: Human chorionic gonadotropin beta-subunit is encoded by at least eight genes arranged in tandem and inverted pairs. Nature 1982, 300(5891):419-422.
55. Fiddes JC, Goodman HM: The cDNA for the beta-subunit of human chorionic gonadotropin suggests evolution of a gene by readthrough into the 3'-untranslated region. Nature 1980, 286(5774):684-687.
56. Venter JC, Adams MD, Myers EW, Li PW, Mural RJ, Sutton GG, Smith HO, Yandell M, Evans CA, Holt RA et al: The sequence of the human genome. Science 2001, 291(5507):1304-1351.
57. Talmadge K, Vamvakopoulos NC, Fiddes JC: Evolution of the genes for the beta subunits of human chorionic gonadotropin and luteinizing hormone. Nature 1984, 307(5946):37-40.
58. Policastro P, Ovitt CE, Hoshina M, Fukuoka H, Boothby MR, Boime I: The beta subunit of human chorionic gonadotropin is encoded by multiple genes. Journal of Biological Chemistry 1983, 258(19):11492-11499.
59. Chassin D, Laurent A, Janneau JL, Berger R, Bellet D: Cloning of a new member of the insulin gene superfamily (INSL4) expressed in human placenta. Genomics 1995, 29(2):465-470.
60. Bellet D, Lavaissiere L, Mock P, Laurent A, Sabourin JC, Bedossa P, Le Bouteiller P, Frydman R, Troalen F, Bidart JM: Identification of pro-EPIL and EPIL peptides translated from insulin-like 4 (INSL4) mRNA in human placenta. J Clin Endocrinol Metab 1997, 82(9):3169-3172.
61. Bieche I, Laurent A, Laurendeau I, Duret L, Giovangrandi Y, Frendo JL, Olivi M, Fausser JL, Evain-Brion D, Vidaud M: Placenta-specific INSL4 expression is mediated by a human endogenous retrovirus element. Biol Reprod 2003, 68(4):1422-1429.
62. Janneau JL, Maldonado-Estrada J, Tachdjian G, Miran I, Motte N, Saulnier P, Sabourin JC, Cote JF, Simon B, Frydman R et al: Transcriptional expression of genes involved in cell invasion and migration by normal and tumoral trophoblast cells. J Clin Endocrinol Metab 2002, 87(11):5336-5339.
63. Brandt B, Roetger A, Bidart JM, Packeisen J, Schier K, Mikesch JH, Kemming D, Boecker W, Yu D, Buerger H: Early placenta insulin-like growth factor (pro-EPIL) is overexpressed and secreted by c-erbB-2-positive cells with high invasion potential. Cancer Res 2002, 62(4):1020-1024.
64. Macaulay EC, Weeks RJ, Andrews S, Morison IM: Hypomethylation of functional retrotransposon-derived genes in the human placenta. Mamm Genome 2011, 22(11-12):722-735.
65. Mock P, Frydman R, Bellet D, Diawara DA, Lavaissiere L, Troalen F, Bidart JM: Pro-EPIL forms are present in amniotic fluid and maternal serum during normal pregnancy. J Clin Endocrinol Metab 1999, 84(6):2253-2256.
66. Bellet D, Lazar V, Bieche I, Paradis V, Giovangrandi Y, Paterlini P, Lidereau R, Bedossa P, Bidart JM, Vidaud M: Malignant transformation of nontrophoblastic cells is associated with the expression of chorionic gonadotropin beta genes normally transcribed in trophoblastic cells. Cancer Res 1997, 57(3):516-523.
67. Marcillac I, Troalen F, Bidart JM, Ghillani P, Ribrag V, Escudier B, Malassagne B, Droz JP, Lhomme C, Rougier P et al: Free human chorionic gonadotropin beta subunit in gonadal and nongonadal neoplasms. Cancer Res 1992, 52(14):3901-3907.
68. Mock P, Frydman R, Bellet D, Chassin D, Bischof P, Campana A, Bidart JM: Expression of pro-EPIL peptides encoded by the insulin-like 4 (INSL4) gene in chromosomally abnormal pregnancies. J Clin Endocrinol Metab 2000, 85(10):3941-3944.
69. Wapner R, Thom E, Simpson JL, Pergament E, Silver R, Filkins K, Platt L, Mahoney M, Johnson A, Hogge WA et al: First-trimester screening for trisomies 21 and 18. N Engl J Med 2003, 349(15):1405-1413.

## Claims

1. Method for the selection of a serum biomarker of epigenetic alterations, particularly of global hypomethylation, said method comprising the steps of:
a) selecting a gene which is expressed as a protein or a peptide in a cancer and/or a placental cell, preferably both in a cancer cell and in a placental cell, and whose expression in said cell is due to the global hypomethylation of the cell, particularly of the expression regulation system/promoter of said gene;
b) optionally, determining the significant presence or the increase of the protein, or a specific fragment thereof, encoded by said gene selected in step a) in a serum or plasma sample of a patient known to exhibit a cancer or a placental-related pathology, preferably compared to a healthy patient serum or plasma sample; and
c) selecting said protein, or a specific fragment thereof, encoded by said gene as a serum biomarker of epigenetic alterations, particularly of global hypomethylation, whether this gene satisfy the step a) and, optionally, step b) selection.

2. A method for diagnosing pathology associated to epigenetic alterations, particularly to global hypomethylation, of cell in a patient comprising the steps of:
i) selecting at least one serum biomarker of epigenetic alterations, particularly of global hypomethylation, selected by the method of claim 1, whose cell specific expression is due to the global hypomethylation of the cell, particularly of the system/promoter which regulates its expression;
ii) optionally, determining the methylation status, preferably the global hypomethylation of the cell, particularly the hypomethylation of said regulation system, or part thereof, in a sample from said patient;
iii) determining the presence or the level of said biomarker selected in step a) in a serum or plasma sample of said patient,
wherein the significant presence or the increase of said biomarker in said patient sample, preferably compared with said biomarker level found for healthy patient serum or plasma sample, is indicative of said pathology.

3. The method according to claim 2, wherein said pathology associated to epigenetic alteration is a cancer or a placental-related pathology.

4. A method of detecting a predisposition to, or the incidence of, placental-related pathology or cancer pathology in a patient which comprises:
i) selecting at least one serum biomarker of epigenetic alterations particularly of global hypomethylation selected by the method of claim 1;
ii) determining the presence or the level of said biomarker selected in step a) in a serum or plasma sample of said patient,
wherein the significant presence or the increase of said biomarker in said patient sample, preferably compared with the level of said biomarker found for healthy patient serum or plasma sample, is indicative of a predisposition to, or the incidence of, placental-related pathology or cancer.

5. The method according to one of claims 2 to 4, wherein said expression regulation system is selected from promoter or retrotransposon expression regulation system.

6. The method according to one of claims 2 to 5, wherein said at least one serum biomarker of epigenetic alterations, particularly of global hypomethylation, is selected from the group consisting of the INSL4 (pro-EPIL) and hCGβ type II biomarker.

7. The method according to one of claims 2 to 6, wherein in step ii) or iii), the determination of the presence or the level of said biomarker in a serum or plasma sample of said patient is carried out by the use of antibody specifically directed against the selected serum biomarker of epigenetic alterations, particularly of global hypomethylation.

8. A method for diagnosing pathology associated to epigenetic alterations, particularly to global hypomethylation, of cell in a patient, said method comprises the step of:
a) specifically detecting or quantifying the presence of the pro-EPIL peptide and/or of HCGβ type II subunit in a serum or plasma sample from said patient susceptible of containing pro-EPIL and/or HCGβ subunits type II, by a method which implements the use of a polyclonal or a monoclonal antibody (mAb) specifically directed to said pro-EPIL peptide, or to a specific fragment thereof, and/or the use of a polyclonal antibody or mAb specifically directed to said HCGβ type II subunit, or to a specific fragment thereof.

9. A method for detecting a predisposition to, or the incidence of, placental-related pathology or cancer pathology in a patient, said method comprises the step of:
a) specifically detecting or quantifying the presence of the pro-EPIL peptide and/or of HCGβ type II subunit peptide, or specific fragment thereof, in a serum or plasma sample from said patient susceptible of containing pro-EPIL and/or HCGβ subunits type II peptide, by a method which implements the use of a polyclonal antibody or a mAb specifically directed to said pro-EPIL peptide, or to a specific fragment thereof, and/or the use of a polyclonal antibody or a mAb specifically directed to said HCGβ type II subunit, or to a specific fragment thereof.

10. The method according to one of claims 8 and 9, wherein said antibody specifically directed to said pro-EPIL peptide or to said HCGβ type II subunit peptide is selected from the group consisting of:
a) polyclonal, monoclonal chimeric or humanized antibodies able to selectively bind, or which selectively bind to an epitope-containing a pro-EPIL polypeptide comprising a contiguous span of at least 9 to 10 amino acids of a pro-EPIL fragment, particularly of a fragment of at least the chain A, B or C of the human pro-EPIL; or
b) polyclonal, monoclonal chimeric or humanized antibodies able to selectively bind HCGβ type II subunit, or a specific fragment thereof, preferably able to selectively bind a discontinuous epitope that comprises region 1-7 with a lysine and a proline residue at position 2 and 4 respectively and region 82-92 of HCGβ, or an anti HCGβ type II-mAb specific binding fragment thereof.

11. The method of claim 10, wherein this antibody specifically directed to said HCGβ type II subunit peptide is selected from the group consisting of:
- the mAb FBT-11-II produced by the hybridoma deposited with the CNCM (Collection Nationale de Cultures de Microorganismes, Institut Pasteur, 25 rue du Docteur Roux, F-75724 PARIS Cedex 15) on March 9, 2010 under the number I-4281;
- the mAb FBT-11 produced by the hybridoma deposited with the CNCM on October 3, 1985 under the number I-489;
- a recombinant mAb having a sequence comprising at least the 6 CDRs (Complementary Determining Region) of the mAb FBT-11-II produced by the hybridoma deposited under the number I-4281or at least the 6 CDRs of the the mAb FBT-11 produced by the hybridoma deposited under the number I-489;
- a mAb specifically directed to a discontinuous epitope that comprises region 1-7 with a lysine and a proline residue at position 2 and 4 respectively and region 82-92 of HCGβ, wherein this antibody is produced by an hybridoma obtained from a mouse which has been prior immunized with an antigen comprising at least the fragments 1-7 with a lysine and a proline residue at position 2 and 4 respectively and 82-92 of HCGβ, said hybridoma being selected based on the capability of its secreted mAb to specifically recognizing the fragments 1-7 with a lysine and a proline residue at position 2 and 4 of the HCGβ type II; and
- a HCGβ type II-binding fragment thereof.

12. The method of one of claims 2 to 11, wherein the determination of the presence or the level of said biomarker in a serum or plasma sample of said patient is carried out by immunoassay, preferably by ELISA immunoassay or radioimmunoassay in blood serum or plasma.

13. A kit for diagnosing pathology associated to epigenetic alteration, particularly to global hypomethylation, of cell in a patient, said kit comprising:
a) an antibody directed specifically against the pro-EPIL peptide, or specific fragment thereof; or/and
b) an antibody directed specifically against the HCGβ-type II subunit peptide, or specific fragment thereof.

14. The kit according to claim 13, wherein said pathology associated to epigenetic alteration is a cancer or a placental-related pathology.

15. A kit for detecting a predisposition to, or the incidence of, placental-related pathology or cancer pathology in a patient, said kit comprising:
a) an antibody directed specifically against the pro-EPIL peptide, or specific fragment thereof; or/and
b) an antibody directed specifically against the HCGβ-type II subunit peptide, or specific fragment thereof.


SEQUENCE LISTING

<110>  UNIVERSITE PARIS DESCARTES

<120>  Method for the selection of serum biomarkers of epigenetic
alterations,
       particularly of global hypomethylation and their uses

<130>  69841 D30805

<160>  15

<170>  PatentIn version 3.3

<210>  1
<211>  139
<212>  PRT
<213>  Homo sapiens

<400>  1

Met Ala Ser Leu Phe Arg Ser Tyr Leu Pro Ala Ile Trp Leu Leu Leu
1               5                   10                  15

Ser Gln Leu Leu Arg Glu Ser Leu Ala Ala Glu Leu Arg Gly Cys Gly
            20                  25                  30

Pro Arg Phe Gly Lys His Leu Leu Ser Tyr Cys Pro Met Pro Glu Lys
        35                  40                  45

Thr Phe Thr Thr Thr Pro Gly Gly Trp Leu Leu Glu Ser Gly Arg Pro
    50                  55                  60

Lys Glu Met Val Ser Thr Ser Asn Asn Lys Asp Gly Gln Ala Leu Gly
65                  70                  75                  80

Thr Thr Ser Glu Phe Ile Pro Asn Leu Ser Pro Glu Leu Lys Lys Pro
                85                  90                  95

Leu Ser Glu Gly Gln Pro Ser Leu Lys Lys Ile Ile Leu Ser Arg Lys
            100                 105                 110

Lys Arg Ser Gly Arg His Arg Phe Asp Pro Phe Cys Cys Glu Val Ile
        115                 120                 125

Cys Asp Asp Gly Thr Ser Val Lys Leu Cys Thr
    130                 135

<210>  2
<211>  22
<212>  PRT
<213>  Homo sapiens

<400>  2

Met Ala Ser Leu Phe Arg Ser Tyr Leu Pro Ala Ile Trp Leu Leu Leu
1               5                   10                  15

Ser Gln Leu Leu Arg Glu
            20

<210>  3
<211>  30
<212>  PRT
<213>  Homo sapiens

<400>  3

Ser Leu Ala Ala Glu Leu Arg Gly Cys Gly Pro Arg Phe Gly Lys His
1               5                   10                  15

Leu Leu Ser Tyr Cys Pro Met Pro Glu Lys Thr Phe Thr Thr
            20                  25                  30

<210>  4
<211>  50
<212>  PRT
<213>  Homo sapiens

<400>  4

Leu Glu Ser Gly Arg Pro Lys Glu Met Val Ser Thr Ser Asn Asn Lys
1               5                   10                  15

Asp Gly Gln Ala Leu Gly Thr Thr Ser Glu Phe Ile Pro Asn Leu Ser
            20                  25                  30

Pro Glu Leu Lys Lys Pro Leu Ser Glu Gly Gln Pro Ser Leu Lys Lys
        35                  40                  45

Ile Ile
    50

<210>  5
<211>  25
<212>  PRT
<213>  Homo sapiens

<400>  5

Ser Gly Arg His Arg Phe Asp Pro Phe Cys Cys Glu Val Ile Cys Asp
1               5                   10                  15

Asp Gly Thr Ser Val Lys Leu Cys Thr
            20                  25

<210>  6
<211>  617
<212>  DNA
<213>  Homo sapiens

<400>  6
agtctggagc ccagaaggga cacaccagca cagtctggta ggctacagca gcaagtctct      60

aaagaaaggc tgagaacacc cagaacagga gagttcaggt ccaggatggc cagcctgttc     120

cggtcctatc tgccagcaat ctggctgctg ctgagccaac tccttagaga aagcctagca     180

gcagagctga ggggatgtgg tccccgattt ggaaaacact tgctgtcata ttgccccatg     240

cctgagaaga cattcaccac caccccagga gggtggctgc tggaatctgg acgtcccaaa     300

gaaatggtgt caacctccaa caacaaagat ggacaagcct taggtacgac atcagaattc     360

attcctaatt tgtcaccaga gctgaagaaa ccactgtctg aagggcagcc atcattgaag     420

aaaataatac tttcccgcaa aaagagaagt ggacgtcaca gatttgatcc attctgttgt     480

gaagtaattt gtgacgatgg aacttcagtt aaattatgta catagtagag taatcatgga     540

ctggacatct catccattct catatgtatt ctcaatgaca aattcactga tgcccaatta     600

aatgattgct gtttaaa                                                    617


<210>  7
<211>  417
<212>  DNA
<213>  Homo sapiens

<400>  7
atggccagcc tgttccggtc ctatctgcca gcaatctggc tgctgctgag ccaactcctt      60

agagaaagcc tagcagcaga gctgagggga tgtggtcccc gatttggaaa acacttgctg     120

tcatattgcc ccatgcctga gaagacattc accaccaccc caggagggtg gctgctggaa     180

tctggacgtc ccaaagaaat ggtgtcaacc tccaacaaca aagatggaca agccttaggt     240

acgacatcag aattcattcc taatttgtca ccagagctga agaaaccact gtctgaaggg     300

cagccatcat tgaagaaaat aatactttcc cgcaaaaaga gaagtggacg tcacagattt     360

gatccattct gttgtgaagt aatttgtgac gatggaactt cagttaaatt atgtaca        417


<210>  8
<211>  66
<212>  DNA
<213>  Homo sapiens

<400>  8
atggccagcc tgttccggtc ctatctgcca gcaatctggc tgctgctgag ccaactcctt      60

agagaa                                                                 66


<210>  9
<211>  90
<212>  DNA
<213>  Homo sapiens

<400>  9
agcctagcag cagagctgag gggatgtggt ccccgatttg gaaaacactt gctgtcatat      60

tgccccatgc ctgagaagac attcaccacc                                    90


<210>  10
<211>  150
<212>  DNA
<213>  Homo sapiens

<400>  10
ctggaatctg gacgtcccaa agaaatggtg tcaacctcca acaacaaaga tggacaagcc      60

ttaggtacga catcagaatt cattcctaat ttgtcaccag agctgaagaa accactgtct     120

gaagggcagc catcattgaa gaaaataata                                      150


<210>  11
<211>  75
<212>  DNA
<213>  Homo sapiens

<400>  11
agtggacgtc acagatttga tccattctgt tgtgaagtaa tttgtgacga tggaacttca      60

gttaaattat gtaca                                                       75

<210>  12
<211>  880
<212>  DNA
<213>  homo sapiens

<220>
<221>  misc_feature
<223>  Chorionic gonadotropin, beta polypeptide 5 (CGB5)

<400>  12
   tccagcactt tgctcgggtc acggcctcct cctggctccc aggaccccac cataggcaga      60
   ggcaggcctt cctacaccct actccctgtg cctccaggct cgactagtcc ctagcactcg     120
   acgactgagt ctctgagatc acttcaccgt ggtctccgcc tcacccttgg cgctggacca     180
   gtgagaggag agggctgggg cgctccgctg agccactcct gcgcccccct ggccttgtct     240
   acctcttgcc ccccgaaggg ttagtgtcga gctcacccca gcatcctaca acctcctggt     300
   ggccttgccg cccccacaac cccgaggtat aaagccaggt acacgaggca ggggacgcac     360
   caaggatgga gatgttccag gggctgctgc tgttgctgct gctgagcatg ggcgggacat     420
   gggcatccaa ggagccgctt cggccacggt gccgccccat caatgccacc ctggctgtgg     480
   agaaggaggg ctgccccgtg tgcatcaccg tcaacaccac catctgtgcc ggctactgcc     540
   ccaccatgac ccgcgtgctg cagggggtcc tgccggccct gcctcaggtg gtgtgcaact     600
   accgcgatgt gcgcttcgag tccatccggc tccctggctg cccgcgcggc gtgaaccccg     660
   tggtctccta cgccgtggct ctcagctgtc aatgtgcact ctgccgccgc agcaccactg     720
   actgcggggg tcccaaggac caccccttga cctgtgatga cccccgcttc caggactcct     780
   cttcctcaaa ggcccctccc cccagccttc caagtccatc ccgactcccg gggccctcgg     840
   acaccccgat cctcccacaa taaaggcttc tcaatccgca                           880

<210>  13
<211>  165
<212>  PRT
<213>  homo sapiens

<220>
<221>  misc_feature
<223>  Chorionic gonadotropin, beta polypeptide 5 (CGB5)

<400>  13

Met Glu Met Phe Gln Gly Leu Leu Leu Leu Leu Leu Leu Ser Met Gly
1               5                   10                  15

Gly Thr Trp Ala Ser Lys Glu Pro Leu Arg Pro Arg Cys Arg Pro Ile
            20                  25                  30

Asn Ala Thr Leu Ala Val Glu Lys Glu Gly Cys Pro Val Cys Ile Thr
        35                  40                  45

Val Asn Thr Thr Ile Cys Ala Gly Tyr Cys Pro Thr Met Thr Arg Val
    50                  55                  60

Leu Gln Gly Val Leu Pro Ala Leu Pro Gln Val Val Cys Asn Tyr Arg
65                  70                  75                  80

Asp Val Arg Phe Glu Ser Ile Arg Leu Pro Gly Cys Pro Arg Gly Val
                85                  90                  95

Asn Pro Val Val Ser Tyr Ala Val Ala Leu Ser Cys Gln Cys Ala Leu
            100                 105                 110

Cys Arg Arg Ser Thr Thr Asp Cys Gly Gly Pro Lys Asp His Pro Leu
        115                 120                 125

Thr Cys Asp Asp Pro Arg Phe Gln Asp Ser Ser Ser Ser Lys Ala Pro
    130                 135                 140

Pro Pro Ser Leu Pro Ser Pro Ser Arg Leu Pro Gly Pro Ser Asp Thr
145                 150                 155                 160

Pro Ile Leu Pro Gln
                165


<210>  14
<211>  7
<212>  PRT
<213>  Artificial

<220>
<223>  Synthetic 7-mer peptides corresponding to residues 1 through 7 of
       HCGbeta encoded by type II genes beta3, beta5 and beta8

<400>  14

Ser Lys Glu Pro Leu Arg Pro
1               5


<210>  15
<211>  7
<212>  PRT

<213>  artificial

<220>
<223>  Synthetic 7-mer peptides corresponding to residues 1 through 7 of
      HCGbeta encoded by type I gene beta7

<400>  15

Ser Arg Glu Met Leu Arg Pro
1               5